# EUROPEAN PATENT SPECIFICATION

(11) **EP 2 630 939 B1**
(45) Date of publication and mention of the grant of the patent: **13.05.2020**
(21) Application number: 11834321.9
(22) Date of filing: 17.10.2011
(51) Int. Cl.: A61F 13/49, A61F 13/15, A61F 13/53

(54) **ABSORBENT ARTICLE**
SAUGFÄHIGER ARTIKEL
ARTICLE ABSORBANT

(30) Priority: 14.10.2011 JP 2011226510; 18.10.2010 JP 2010233376
(43) Date of publication of application: 28.08.2013
(73) Proprietor: Kao Corporation, Chuo-Ku Tokyo 103-8210 (JP)
(72) Inventor: KIKKAWA, Muneyoshi, Haga-gun Tochigi 321-3497 (JP); SATO, Nobuya, Haga-gun Tochigi 321-3497 (JP)
(74) Representative: Vossius & Partner Patentanwälte Rechtsanwälte mbB
(86) International application number: PCT/JP2011/073841
(87) International publication number: WO 2012/053482

(56) References cited:
- JP-A- 3 236 838
- JP-A- 2007 202 661
- JP-A- 2010 115 352
- JP-A- 2010 529 900
- US-A- 5 368 926
- US-A1- 2008 312 621

## Description

### Technical Field

The present invention relates to an absorbent article such as a disposable diaper, an absorbent pad, and a sanitary napkin.

### Background Art

There has been known the above type of absorbent articles including a liquid permeable topsheet which forms a skin-facing face, a liquid impermeable backsheet which forms a non-skin-facing face, and an absorbent member arranged between both the sheets. Further, there has been known the absorbent member which is structured to include an absorbent core containing cellulose fibers such as pulp and/or absorbent polymer and a core wrap sheet which covers the absorbent core. The core wrap sheet functions as a sheet for receiving an absorbent core forming material such as absorbent polymer when manufacturing the absorbent member and functions to form a shape as wrapping the absorbent core after manufacturing. Traditionally, a permeable sheet such as nonwoven and paper has been used as the core wrap sheet.

Traditionally, numerous attempts have been performed as targeting improvement of fit against a body of a wearer and liquid absorbability for such a kind of absorbent articles. Examples of an effective method to improve fit include a method to lessen a thickness of an absorbent member (absorbent core) as decreasing a usage amount of cellulose fibers being bulky absorbent core forming materials. When the absorbent member is thinned, flexibility of the absorbent member is improved and fit is improved thereby. For example, Patent Literature 1 discloses an absorbent article having the abovementioned structure with an absorbent core structured to include a trapping system in which cellulose fibers are not substantially contained.

US 2008/312621 (A1) relates to a disposable absorbent article, such as a diaper, with an improved acquisition system. US 5 368 926 (A) relates to fluid accepting, transporting, and retaining structures, and more specifically, to nonwoven webs capable of both intrafiber and interfiber fluid transport.

### Citation List

### Patent Literature

Patent Literature 1: U.S. 2008/0021426A1

### Summary of Invention

### Technical Problem

With the absorbent article including a thin-type absorbent core which does not substantially contain cellulose fibers as disclosed in Patent Literature 1, a distance between the absorbent core forming material such as absorbent polymer absorbing and retaining bodily discharge liquid and a skin of a wearer is short while wearing the absorbent article compared to an absorbent article including a relatively thick absorbent core which is mainly structured with cellulose fibers. Accordingly, liquid remaining is more likely to occur at a topsheet which forms a skin-facing face and a dry feeling at the topsheet is more likely to be decreased. Thus, there is a fear to cause a trouble such as a skin rash.

### Solution to Problem

The present invention is directed to an absorbent article including: a topsheet which forms a skin-facing face; a backsheet which forms a non-skin-facing face; and an absorbent member which is arranged between both the sheets, in which the absorbent member is structured to include an absorbent core which contains absorbent polymer without substantially containing hydrophilic fibers, an upper core wrap sheet which covers a skin-facing face of the absorbent core, and a lower core wrap sheet which covers a non-skin-facing face of the absorbent core, and the upper core wrap sheet includes a thick region having a larger thickness than the lower core wrap sheet.

### Advantageous Effects of Invention

The present invention provides an absorbent article having excellent fit and a dry feeling at a skin-facing face.

### Brief Description of Drawings

[Fig. 1] Fig. 1 is a view illustrating a disposable diaper which is an embodiment of an absorbent article of the present invention being a plane view at a skin-facing face side (topsheet side) schematically illustrating a planarly-expanded state with respective elastic members extended.
[Fig. 2] Fig. 2 is a sectional view schematically illustrating a section (a section in a lateral direction) along line I-I of Fig. 1.
[Fig. 3] Fig. 3 is a sectional view schematically illustrating a section (a section in the lateral direction) along line II-II of Fig. 1.
[Fig. 4] Figs. 4(a) and 4(b) are sectional views schematically illustrating sections in the lateral direction respectively of other embodiments of an absorbent member according to the present invention.
[Fig. 5] Figs. 5(a) to 5(d) are sectional views further illustrating sections schematically in the lateral direction respectively of other embodiments of the absorbent member according to the present invention.
[Fig. 6] Fig. 6(a) is a perspective view illustrating an example of a concave-convex region of an upper core wrap sheet according to the present invention and Fig. 6(b) is a sectional view schematically illustrating a section along line III-III of Fig. 6(a).
[Fig. 7] Fig. 7(a) is a perspective view illustrating another example of the concave-convex region of the upper core wrap sheet according to the present invention and Fig. 7(b) is a sectional view schematically illustrating a section along line IV-IV of Fig. 7(a).
[Fig. 8] Fig. 8 is a schematic view illustrating a method of measuring a sheet hydrophilic degree.

### Description of Embodiments

The present invention relates to an absorbent article having excellent fit and a dry feeling at a skin-facing face.

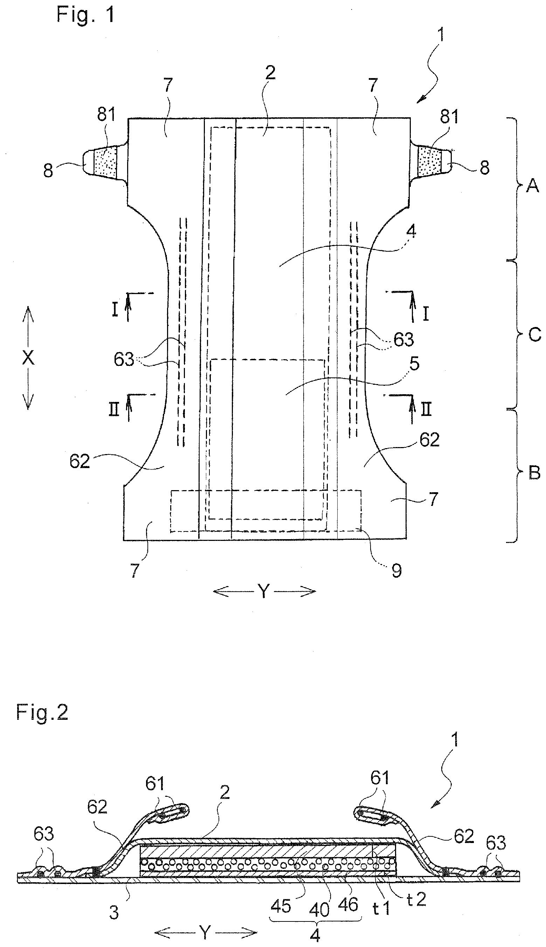

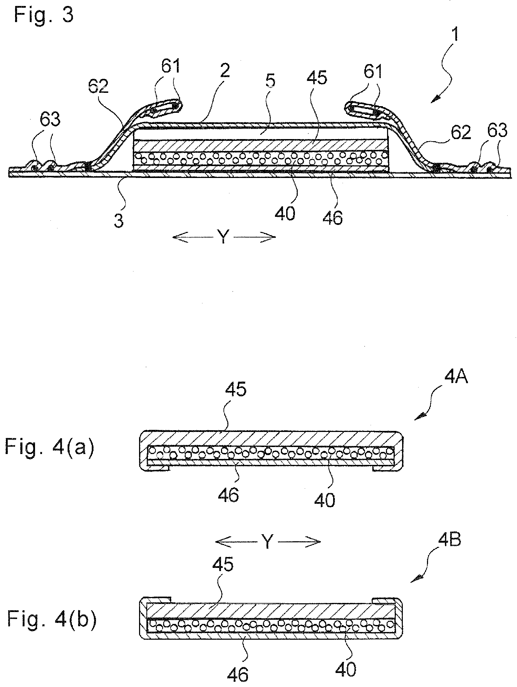

In the following, an absorbent article of the present invention will be described with reference to the drawings based on a disposable diaper being a preferred embodiment thereof. As illustrated in Figs. 1 and 2, a diaper 1 of the present embodiment being a so-called open-style type disposable diaper is provided with a liquid permeable topsheet 2 which forms a skin-facing face, a backsheet 3 being liquid impermeable or water-repellent (hereinafter, called collectively being liquid impermeable) which forms a non-skin-facing face, and an absorbent member 4 which is arranged between both the sheets 2, 3 and is formed substantially being elongated. Each of the topsheet 2, the backsheet 3, and the absorbent member 4 has a shape elongated in one direction X. The topsheet 2 and backsheet 3 have larger dimensions than the absorbent member 4 respectively and are extended outward from a periphery of the absorbent member 4. As illustrated in Figs. 2 and 3, the topsheet 2 has a dimension in a lateral direction Y being smaller than a dimension of the backsheet 3 in the lateral direction Y.

As illustrated in Fig. 1, the diaper 1 has, along a longitudinal direction X, a rear section A adapted to be worn about the back of a wearer, a front section B adapted to be worn about the front of a wearer, and a crotch section C adapted to be worn about the crotch of a wearer. The crotch section C is located at a center part of the diaper 1 in the longitudinal direction X. Both side edges of the crotch section C is inwardly curved to be like an arc shape and the diaper 1 has a shape like a sandglass as a center part in the longitudinal direction X being narrowed inwardly in a plane view as Fig. 1.

In the present specification, the longitudinal direction denotes a direction along a long side of an absorbent article (disposable diaper) or a structural member thereof (e.g., absorbent core) and the lateral direction denotes a direction being perpendicular to the longitudinal direction. In the drawings, a direction indicated by reference X denotes the longitudinal direction of the diaper 1 (absorbent member 4) and a direction indicated by reference Y denotes the lateral direction of the diaper 1 (absorbent member 4). Further, the skin-facing face denotes a face of an absorbent article (disposable diaper) or a structural member thereof facing to a skin side of a wearer while the absorbent article (disposable diaper) is worn. The non-skin-facing face denotes a face of the absorbent article (disposable diaper) or the structural member thereof facing to a side (clothing side) being opposite to the skin side while the absorbent article (disposable diaper) is worn.

As illustrated in Figs. 1 to 3, a side sheet 62 to which an elastic member 61 is fixed at one side edge portion thereof in an extended state is arranged along the longitudinal direction X at each of both side portions of the diaper 1, so that a pair of standing gathers is formed at the crotch section C while worn. Further, an elastic member 63 is arranged along the longitudinal direction X at each of right and left leg portions arranged around legs of a wearer, so that a pair of leg gathers is formed at the leg portions owing to contraction of the elastic members 63 while worn. As illustrated in Fig. 2 and 3, the pair of side sheets 62, 62, the topsheet 2, the absorbent member 4, the elastic members 63, and the backsheet 3 are joined with known joining means such as hot-melt type adhesive.

Further, as illustrated in Fig. 1, a pair of fastening tapes 8, 8 is arranged at both side edge portions of the rear section A of the diaper 1 along the longitudinal direction X. More specifically, side flaps 7, 7 outwardly extended in the lateral direction Y from both side edge portions of the absorbent member 4 along the longitudinal direction X are formed at both side portions of the rear section A and the front section B respectively along the longitudinal direction X. The fastening tapes 8 are attached to the respective side flaps 7 as being outwardly extended in the lateral direction Y. A fastening portion 81 formed of a male member of a mechanical Hook-and-Loop fastener is attached to the fastening tape 8.

Further, a fastened region 9 formed of a female member of the mechanical Hook-and-Loop fastener is formed at the non-skin-facing face of the front section B of the diaper 1. The fastened region 9 is formed by joining and fixing the female member of the mechanical Hook-and-Loop fastener to the non-skin-facing face of the backsheet 3 with known joining means (e.g., adhesive or heat seal) and the fastening portion 81 of the fastening tape 8 can be fastened thereto as being detachably attachable.

In the following, the absorbent member 4 will be described in detail. As illustrated in Figs. 2 and 3, the absorbent member 4 is structured to include an absorbent core 40, an upper core wrap sheet 45 which covers a skin-facing face of the absorbent core 40 (forms the skin-facing face of the absorbent member 4), and a lower core wrap sheet 46 which covers a non-skin-facing face of the absorbent core (forms the non-skin-facing face of the absorbent member 4). As illustrated in Fig. 1, the absorbent member 4 (absorbent core 40) has a rectangle shape elongated in one direction (the longitudinal direction X of the diaper 1) and is formed as being continuously extended from a vicinity of an end in the longitudinal direction of the front section B of the diaper 1 to a vicinity of an end in the longitudinal direction of the rear section A via the crotch section C. The upper core wrap sheet 45 and the lower core wrap sheet 46 have the same shape and the same dimensions in plane view with the same lengths in the longitudinal direction X and the lateral direction Y.

The absorbent core 40 contains absorbent polymer and does not contain hydrophilic fibers substantially. Here, in addition to a case that the absorbent core does not contain hydrophilic fibers at all, "substantial non-containing of hydrophilic fibers" includes a case that a minute amount of hydrophilic fibers are contained, specifically, a case that hydrophilic fiber content of the absorbent core is 5% or less by mass. Here, fibers having hydrophilic surfaces may be used as the hydrophilic fibers and examples thereof include cellulose fibers and synthetic fibers on which a hydrophilicity process is performed with a surface acting agent or the like as needed. Examples of the cellulose fibers include natural cellulose fibers being wood pulp such as softwood kraft pulp and hardwood kraft pulp and non-wood pulp such as cotton pulp and straw pulp, and regenerated cellulose fibers such as rayon and cupra. Further, examples of the synthetic fibers include hydrophilic synthetic fibers such as polyvinyl alcohol fibers and polyacrylonitrile fibers, polyethylene terephthalate (PET) fibers, polyethylene (PE) fibers, polypropylene (PP) fibers, and polyester fibers. Since the absorbent core containing absorbent polymer without substantially containing hydrophilic fibers as described above is thin while retaining practically sufficient liquid absorbability, excellent fit against a body of a wearer is obtained with high flexibility.

The absorbent core 40 is structured mainly with absorbent polymer. Content of absorbent polymer is 95% or more by mass against the total mass of the absorbent core 40 preferably and is in a range of 99% to 100% by mass more preferably. Various types traditionally used in the technical field may be used as the absorbent polymer without specific limitations. Examples thereof include sodium polyacrylate, (acrylic acid-vinyl alcohol) copolymer, crosslinked sodium polyacrylate, (starch-acrylic acid) graft polymer, (isobutylene-maleic acid anhydride) copolymer and saponification thereof, potassium polyacrylate, and cesium polyacrylate. Here, one type may be used solely or two or more types may be used as being mixed. Although a particle type is used as the absorbent polymer normally, a fiber type may be used as well. In view of difference in shape, particle types of the absorbent polymer include an indeterminate type, a block type, a barrel type, a particle aggregation type, and a ball type. Here, any type thereof may be used.

A basis weight of the absorbent core 40 is appropriately set in accordance with usage application and the like of an absorbent article (disposable diaper) to which the absorbent core 40 is assembled. For example, in a case that the diaper 1 is a paper diaper for an infant (young month age infant), from a viewpoint of balance among thinning, flexibility and liquid absorbability, it is preferable that the basis weight of the absorbent core 40 is in a range of 80 to 400 g/m², and particularly, in a range of 150 to 300 g/m². When the absorbent core 40 is made only of absorbent polymer, the basis weight of the absorbent core 40 is the same as a dispersal amount of the absorbent polymer.

In the present embodiment, in consideration of that absorbent polymer is contained and hydrophilic fibers are not substantially contained being a feature of the absorbent core 40, relation between thicknesses of the upper core wrap sheet 45 and the lower core wrap sheet 46 which cover the absorbent core 40 is devised. Specifically, the upper core wrap sheet 45 includes a thick region having a larger thickness than lower core wrap sheet 46. As illustrated in Figs. 2 and 3, the entire area of the upper core wrap sheet 45 is the thick region in the present embodiment.

Normally, an absorbent core in such an absorbent article is structured to include hydrophilic fibers such as cellulose fibers along with absorbent polymer. Here, when hydrophilic fibers is reduced in amount from such a normal absorbent core as targeting thinning and improving of fit and the like, a distance between the absorbent core (absorbent polymer) in a wet state as absorbing and retaining liquid and a skin of a wearer becomes short while wearing the absorbent article compared to that before reducing the hydrophilic fibers. Accordingly, there is a fear to cause a trouble such as a skin rash with decrease of a dry feeling at a topsheet. Further, when the lower core wrap sheet which covers the non-skin-facing face of the absorbent core becomes excessively thick, liquid leakage is more likely to occur as described below. When the thickness of the lower core wrap sheet becomes large, liquid is to be retained in the lower core wrap sheet. Here, since the skin-facing face side of the lower core wrap sheet at which liquid is retained is contacted to absorbent polymer which structures the absorbent core, liquid retained at the skin-facing face side is immobilized finally at the absorbent polymer. However, since liquid retained at the non-skin-facing face side of the lower core wrap sheet remains without being immobilized at the absorbent polymer, liquid leakage is more likely to occur. In the present embodiment, in consideration of the above, the thick region having a larger thickness than the lower core wrap sheet 46 is formed at the upper core wrap sheet 45. With the above, decrease of a dry feeling at the skin-facing face (topsheet 2) of the diaper 1 caused by reduction of hydrophilic fibers is prevented and improvement is achieved in leakage prevention. Thicknesses of core wrap sheets are measured as follows.

### <Method of measuring thickness of core wrap sheet>

First, adhesive contained in a product (diaper) is dissolved by soaking the product (diaper) into cleaning solvent (mixed solvent of butyl acetate and hexane) and the product is carefully disassembled into structural members. The respective structural members are taken out from the cleaning solvent so as not to be stretched and are dried at room temperature. Subsequently, a load of 5.9 Pa is applied to a dried core wrap sheet by placing an acrylic plate having a 50 millimeters square shape (weight: 14. 8 g) thereon. The thickness of the core wrap sheet is measured with a laser type thickness indicator.

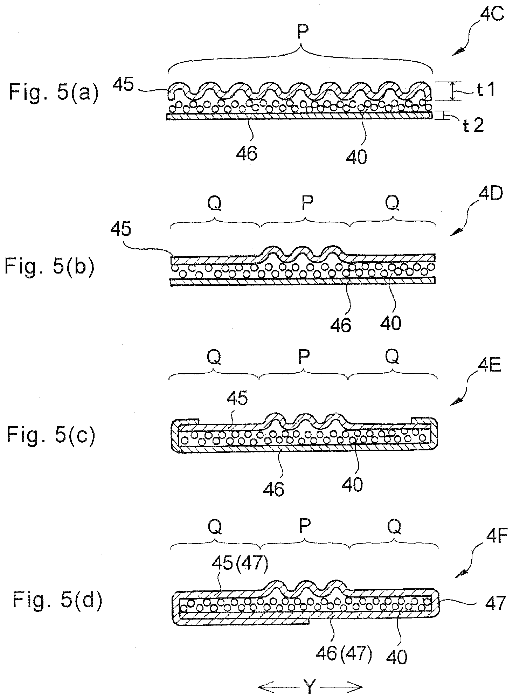

Here, in a case that the upper core wrap sheet includes a concave-convex region where a concave-convex shape is formed as the upper core wrap sheet 45 illustrated in Fig. 5, it is possible that "the thickness of the upper core wrap sheet" being a comparison target to "the thickness of the lower core wrap sheet" denotes an apparent thickness of the concave-convex shape at the concave-convex region (a thickness indicated by a reference t1 in Figs. 5(a), 6(b) and 7(b)) instead of a substantial thickness of the sheet itself. That is, the upper core wrap sheet having the concave-convex region is only required that at least the apparent thickness of the concave-convex region thereof is larger than the thickness of the lower core wrap sheet, so that the concave-convex region is to be the thick region. The apparent thickness of the concave-convex shape is measured with the abovementioned method of measuring a thickness. That is, the apparent thickness of the concave-convex region is measured with a laser type thickness indicator in a state that a load of 5.9 Pa is applied with the acryl plate placed on the concave-convex region of the upper core wrap sheet.

From a viewpoint to reliably produce an operational effect due to the abovementioned thickness ingenuity of the core wrap sheets 45, 46, a ratio (t1/t2) between the thickness t1 (see Figs. 2, 5(a), 6(b), and 7(b)) of the thick region of the upper core wrap sheet 45 and a thickness t2 (see Fig. 2) of the lower core wrap sheet 46 is in a range of 1.5 to 20 preferably and in a rage of 2 to 10 more preferably. That is, it is particularly preferable that the thickness t1 of the thick region of the upper core wrap sheet 45 is twice or more of the thickness t2 of the lower core wrap sheet 46. Further, the thickness t1 of the thick region of the upper core wrap sheet 45 is in a range of 0.15 to 2 mm preferably and in a rage of 0.2 to 1 mm more preferably. The thickness t2 of the lower core wrap sheet 46 is in a range of 0.05 to 0.5 mm and in a range of 0.1 to 0.2 mm more preferably.

Further, the basis weight of the upper core wrap sheet 45 is in a range of 13 to 30 g/m² preferably and in a range of 15 to 20 g/m² more preferably. The basis weight of the lower core wrap sheet 46 is in a range of 7 to 30 g/m² preferably and in a range of 10 to 15 g/m² more preferably. In general, a basis weight and a thickness of a sheet are mutually related. The thickness becomes larger with increase of the basis weight.

The upper core wrap sheet 45 and the lower core wrap sheet 46 are integrated as being mutually joined with joining means (adhesive) such as hot-melt type adhesive. More specifically, both the sheets 45, 46 are mutually joined with adhesive (not illustrated) adherent to the absorbent core 40 (absorbent polymer), so that both the sheets 45, 46 and the absorbent core 40 are integrated with the adhesive.

A sheet which is structured mainly with fibers (a sheet with fiber content preferably being 90% or more by mass) may be adopted respectively as the upper core wrap sheet 45 and the lower core wrap sheet 46. Examples thereof include paper such as crepe paper, nonwoven such as spunbond nonwoven, meltblown nonwoven, thermalbonded nonwoven, needle-punched nonwoven, spanlaced nonwoven and airlaid nonwoven, and a combined sheet with two types or more of the sheets laminated. Not being structured with the respective sheets structuring the combined sheet mutually stuck with adhesive, the combined sheet is preferably to be in one sheet while the respective sheets are integrated with entanglement and/or thermal adhesion of mutual structural fibers of the respective sheets like a laminated body of nonwoven described later. Both the sheets 45, 46 may be the same sheets or different sheets.

It is preferable that each of the upper core wrap sheet 45 and the lower core wrap sheet 46 is formed of a laminated body with one or more of spunbond nonwoven layers and one or more of meltblown nonwoven layers. The laminated body (core wrap sheets 45, 46) is one sheet of nonwoven in which the respective nonwoven layers are integrated as being mutually joined. Not being integrated as being mutually stuck with adhesive, adjacent nonwoven layers in the laminated body are integrated with entanglement and/or thermal adhesion of mutual structural fibers of the respective nonwoven layers. With a laminated body in which adjacent nonwoven layers are mutually stuck with adhesive, liquid is more likely to be collected at a boundary face which exists between nonwoven layers as a boundary between the nonwoven layers. Therefore, it is not exactly ideal for a core wrap sheet according to the present invention.

A spunbond nonwoven layer is a layer formed of spunbond nonwoven which is manufactured with a known spunbond method. A meltblown nonwoven layer is a layer formed of meltblown nonwoven which is manufactured with a known meltblown method. For example, the spunbond nonwoven can be obtained by that thermal-fused synthetic resin spins out from a nozzle, a number of generated filaments are amassed on a conveyer to be a web, and the respective filaments are joined with a process such as thermal compression bonding and mechanical interlacing on the web. Further, for example, meltblown nonwoven can be obtained by forming a self-joining fiber web while melted synthetic resin is blown onto a conveyer from a die chip of an extruding machine with high-speed air-flow. Examples of constituent fibers for spunbond nonwoven and meltblown nonwoven include fibers made solely of polyolefin resin such as polyethylene and polypropylene, polyester resin such as polyethylene terephthalate or the like, or conjugate fibers composed of a plurality of such resins (a sheath/core conjugate type, a side-by-side type or the like).

Specifically preferable examples of the laminated body (core wrap sheets 45, 46) include spunbond-meltblown-spunbond (SMS) nonwoven being nonwoven integrated in a state that one meltblown nonwoven layer is placed between two spunbond nonwoven layers or spunbond-meltblown-meltblown-spunbond (SMMS) nonwoven being nonwoven integrated in a state that two meltblown nonwoven layers are placed between two spunbond nonwoven layers.

In addition to that the upper core wrap sheet 45 is formed of a laminated body of one or more of spunbond nonwoven layers and one or more of meltblown nonwoven layers, it is preferable that a basis weight of the spunbond nonwoven layer of the upper core wrap sheet 45 is larger than that of the meltblown nonwoven layer. Here, "the basis weight" denotes a sum of basis weights of the respective nonwoven layers. For example, in a case that two spunbond nonwoven layers exist in the laminated body (upper core wrap sheet 45) including a case that another nonwoven layer exists between the two spunbond nonwoven layers as well as a case that the two spunbond nonwoven layers are mutually contacted, it denotes a sum of the respective basis weights of the two spunbond nonwoven layers. From a viewpoint of improving a dry feeling at the topsheet 2, it is desired that the upper core wrap sheet 45 placed between the topsheet 2 and the absorbent core 40 has a large free-water retention amount. In general, spunbond nonwoven has a feature of having a large thickness as being bulky and a large free-water retention amount compared to meltblown nonwoven. Therefore, it is preferable for the upper core wrap sheet 45 formed of the laminated body that a basis weight of the spunbond nonwoven layer is relatively large.

Further, in addition to that the lower core wrap sheet 46 is formed of a laminated body of one or more of spunbond nonwoven layers and one or more of meltblown nonwoven layers, it is preferable that a basis weight of the meltblown nonwoven layer of the lower core wrap sheet 46 is larger than that of the spunbond nonwoven layer. Here, "the basis weight" denotes as described above. The lower core wrap sheet 46 is placed between the absorbent core 40 and the backsheet 3. From a viewpoint of leakage prevention, it is preferable that a free-water retention amount of the sheet 46 itself is small. Therefore, it is preferable for the lower core wrap sheet 46 that a basis weight of the spunbond nonwoven layer being superior in free-water retention capability is relatively small by relatively enlarging a basis weight of the meltblown nonwoven layer.

In the upper core wrap sheet 45 formed of the laminated body, a basis weight ratio between the spunbond nonwoven layer and the meltblown nonwoven layer (the former / the latter) is in a range of 5 to 15 preferably and in a range of 7 to 10 more preferably. Further, in the lower core wrap sheet 46 formed of the laminated body, a basis weight ratio between the spunbond nonwoven layer and the meltblown nonwoven layer (the former / the latter) is in a range of 0.05 to 0.2 preferably and in a range of 0.1 to 0.15 more preferably.

It is preferable that the upper core wrap sheet 45 and the lower core wrap sheet 46 are different in hydrophilicity as well as in thickness. More specifically, a hydrophilic degree of the upper core wrap sheet 45 is higher than that of the lower core wrap sheet 46. That is, from a viewpoint that bodily discharge liquid such as urine is quickly permeated to the absorbent core 40 side, it is preferable that the upper core wrap sheet 45 has a relatively high hydrophilic degree. Further, from a viewpoint of leakage prevention, liquid spreading acceleration in the absorbent core 40 and the like, it is preferable that the lower core wrap sheet 46 has hydrophobicity or slight hydrophilicity on the order not to retain free water (bodily discharge liquid). A hydrophilic degree of a sheet can be evaluated by a water passing time measured with a method described below. The shorter the water passing time is, the higher the hydrophilic degree of the sheet is evaluated. The water passing time of the upper core wrap sheet 45 is different from that of the lower core wrap sheet 46. The water passing time of the upper core wrap sheet 45 having a relatively-high hydrophilic degree is shorter than 15 seconds preferably and is shorter than 10 seconds more preferably. The water passing time of the lower core wrap sheet 46 having a relatively-low hydrophilic degree is 15 seconds or longer preferably and is 60 seconds or longer more preferably.

### <Method of measuring sheet hydrophilic degree (water passing time)>

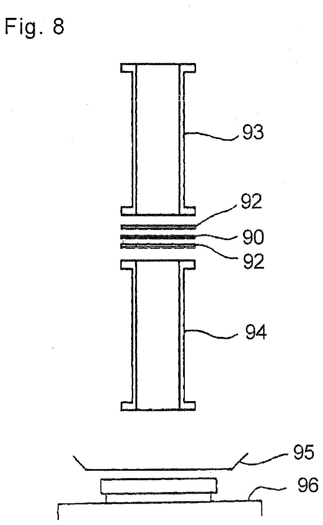

As illustrated in Fig. 8, ion-exchange water of 40 g is supplied to an upper cylinder 93 in a state that a sheet 90 being a measurement target is fixed as being sandwiched by a pair of glass-made cylinders 93, 94 having a diameter of 35 mm via rubber gaskets 92, 92 from upper and lower sides. Measuring a time from supply starting of ion-exchange water until the weight of ion-exchange water accumulated in the lower cylinder 94 becomes 20 g as passing through the sheet 90, the time denotes the water passing time. The weight of the ion-exchange water is measured with an electric balance 96 having a liquid receiving tray 95.

Examples of a method of relatively increasing the hydrophilic degree of the upper core wrap sheet 45 and relatively decreasing the hydrophilic degree of the lower core wrap sheet 46 include a method to perform a hydrophilization process only on the upper core wrap sheet 45 without performing the hydrophilization process on the lower core wrap sheet 46 when both the core wrap sheets 45, 46 are nonwoven. Examples of a method of the hydrophilization process include a method to perform a process (immersing, spraying, gravure coating, printing or the like) on a sheet (nonwoven) with hydrophilization oil solution and a method for kneading hydrophilization oil solution into resin being a material of nonwoven and bleeding out.

The diaper 1 of the present embodiment will be further described. As illustrated in Fig. 1, a sublayer sheet 5 is arranged between the topsheet 2 and the absorbent member 4. The sublayer sheet 5 has a function to reduce a phenomenon (wet-back) that liquid absorbed to the absorbent member 4 returns to the topsheet 2 side by enhancing liquid absorbability of the diaper 1. The sublayer sheet 5 is not arranged to cover the entire area of the skin-facing face (upper core wrap sheet 45) of the absorbent member 4. The sublayer sheet 5 is arranged at a part of the skin-facing face of the absorbent member 4, specifically, only at a region of the skin-facing face of the absorbent member 4 from a vicinity of an end in the longitudinal direction of the front section B to a center part in the longitudinal direction of the absorbent member 4 located at the crotch section C, and is not arranged at the rear section A. The absorbent member 4 and the sublayer sheet 5 may be joined with adhesive such as hot-melt type adhesive therebetween.

From a viewpoint that the operational effects (improvement of a dry feeling at the topsheet, and the like) due to the abovementioned absorbent member 4 are to be reliably produced, it is preferable that the skin-facing face (upper core wrap sheet 45) of the absorbent member 4 in 30% or more of the total area, particularly in a range of 50% to 60%, is not covered with another member like the sublayer sheet 5 which is arranged between the topsheet 2 and the absorbent member 4. When the entire area of the skin-facing face of the absorbent member 4 (100% of the total area of the skin-facing face) is covered with the sublayer sheet 5, there is a fear that the abovementioned ingenuity of the core wrap sheets 45, 46 is not utilized.

A sheet which is structured mainly with hydrophilic fibers (preferably, a sheet with content of the hydrophilic fibers being 90% or more by mass) may be adopted as the sublayer sheet 5. Examples of the sheet include paper, nonwoven, and a web. Fibers having hydrophilic surfaces may be used as the hydrophilic fibers without any specific limitation as long as being capable of forming a sheet with a high degree of freedom of mutual fibers in a wet state. In addition to cellulose fibers, hydrophilic synthetic fibers and synthetic fibers on which a hydrophilicity process is performed as described above, examples of the hydrophilic fibers include denatured (cellulose) fibers disclosed in JP 2010-526632A exemplifying kneaded and/or curled (curly) chemically-stiffened (cellulose) fibers, and kneaded and/or curled (curly) chemically-stiffened bridged cellulose or synthetic polymer fibers. Here, the fiber may be used solely or two or more of the fibers may be used as being mixed.

In addition to hydrophilic fibers, the sublayer sheet 5 may include other constituents such as absorbent polymer and deodorant. A basis weight of the sublayer sheet 5 is in a range of 40 to 300 g/m² preferably and in a range of 60 to 200 g/m² more preferably. Further, a thickness of the sublayer sheet 5 with no load is in a range of 1 to 10 mm preferably and in a range of 3 to 5 mm more preferably.

Materials for forming respective components of the diaper 1 will be descried. A variety types of materials traditionally used in the art may be used as the topsheet 2 and the backsheet 3. The topsheet 2 may adopt a variety of liquid permeable sheet materials such as nonwoven and a perforated film. The backsheet 3 may adopt a variety of materials being non-permeable or water-repellent such as a non-permeable resin film, a permeable resin film with fine holes, nonwoven like water-repellent nonwoven or the like, a laminated body of the above and other sheets, or the like. Further, the side sheet 62 may adopt a material being similar to that of the backsheet 3.

In a case that a resin film is used as the backsheet 3, it is preferable that a permeable resin film (e.g., a resin film with fine holes) is used as the resin film to decrease a damp feeling of a wearer. Particularly, to reduce a damp feeling to the extent possible, it is preferable that higher permeability is provided to the resin film for the backsheet 3. As a method to improve permeability of a resin film with fine holes, traditionally, there have been known a method to stretch the resin film (a method to increase the number of fine holes of the resin film compared to that before being stretched by appropriately adjusting stretching conditions and the like), a method to form fine holes which are slightly larger than normal fine holes, and the like. However, with a resin film having permeability increased with such a traditional method, there is a fear that water-pressure resistance is slightly decreased. In a case that such a resin film with reduced water-pressure resistance is used in the diaper 1 as the backsheet 3, there is a fear of inconvenience that liquid absorbed and retained at the absorbent core 40 leaks to the outside as passing through the backsheet 3 when a body weight of a wearer is applied largely to the diaper 1. In contrast, since liquid is less likely to move to the backsheet 3 side by decreasing a hydrophilic degree of the lower core wrap sheet 46 and applying slight hydrophilicity or hydrophobicity on the order not to retain free water (bodily discharge liquid) to the lower core wrap sheet 46 as described above, the abovementioned fear can be swept away. In particular, when the lower core wrap sheet 46 has hydrophobicity, liquid leakage from the backsheet 3 side can be effectively prevented. The water passing time of the lower core wrap sheet 46 having hydrophobicity is 15 seconds or longer preferably and is 60 seconds or longer more preferably.

The diaper 1 of the present embodiment is used similarly to a known open-style type disposable diaper. In the diaper 1 of the present embodiment, since the absorbent member 4 is structured to include the absorbent core 40 which contains absorbent polymer without substantially containing hydrophilic fibers, it is possible to design thinly compared to a traditional absorbent member which mainly contains hydrophilic fibers such as cellulose fibers. Owing to adopting of such a thin type absorbent member, excellent fit can be obtained against a body of a wearer. There may be a fear that thinning of the absorbent core 40 due to substantial non-usage of hydrophilic fibers causes decrease of a dry feeling at the skin-facing face (topsheet 2). However, in the diaper 1 of the present embodiment, since the thick region being thicker than the lower core wrap sheet 46 is formed at the upper core wrap sheet 45, decrease of a dry feeling at the skin-facing face is less likely to occur and a trouble such as a skin rash can be effectively prevented.

As described above, the absorbent member according to the present invention can be designed thinly. The thickness of the thin absorbent member with no load is in a range of 1 to 5 mm preferably and in a range of 2 to 4 mm more preferably.

Not being limited to the abovementioned embodiment, the absorbent member according to the present invention can adopt a variety of types without departing from the scope of the present invention. Other embodiments of the absorbent member according to the present invention are illustrated in Figs. 4 and 5. Regarding other embodiments described later, description will be performed mainly on structural portions being different from the abovementioned embodiment. The same numeral is given to the similar structural portion and description thereof will not be repeated. Description of the abovementioned embodiment is appropriately applied to structural portions which are not specifically described.

In absorbent members 4A, 4B illustrated in Fig. 4, lengths of the upper core wrap sheet 45 and the lower core wrap sheet 46 in the lateral direction Y are mutually different. In the absorbent member 4A, the upper core wrap sheet 45 is relatively wide in width and the lower core wrap sheet 46 is relatively narrow in width. In the absorbent member 4B, the upper core wrap sheet 45 is relatively narrow in width and the lower core wrap sheet 46 is relatively wide in width. Here, in the absorbent members 4A, 4B, lengths in the longitudinal direction X are the same between the upper core wrap sheet 45 and the lower core wrap sheet 46 respectively.

As illustrated in Fig. 4(a), in the absorbent member 4A, the lower core wrap sheet 46 which is relatively narrow in width covers the entire area of the non-skin-facing face of the absorbent core 40. The upper core wrap sheet 45 which is relatively wide in width covers the entire area of the skin-facing face of the absorbent core 40 and is extended outward in the lateral direction Y from both side edge portions of the absorbent core 40 along the longitudinal direction X. Then, the extended portions are rolled down to the lower side of the lower core wrap sheet 46 which is arranged as being faced to the non-skin-facing face of the absorbent core 40 and cover both the side edge portions of the sheet 46 along the longitudinal direction X. Further, as illustrated in Fig. 4(b), in the absorbent member 4B, the upper core wrap sheet 45 which is relatively narrow in width covers the entire area of the skin-facing face of the absorbent core 40. The lower core wrap sheet 46 which is relatively wide in width covers the entire area of the non-skin-facing face of the absorbent core 40 and is extended outward in the lateral direction Y from both side edge portions of the absorbent core 40 along the longitudinal direction X. Then, the extended portions are rolled up to the upper side of the upper core wrap sheet 45 which is arranged as being faced to the skin-facing face of the absorbent core 40 and cover both the side edge portions of the sheet 45 along the longitudinal direction X. Further, in the absorbent members 4A, 4B, the upper core wrap sheet 45 and the lower core wrap sheet 46 are integrated as being mutually jointed with joining means (adhesive) such as hot-melt type adhesive. According to absorbent articles respectively with the absorbent members 4A, 4B, it is possible to obtain similar effects to the abovementioned embodiment.

In absorbent members 4C to 4F illustrated in Fig. 5, the upper core wrap sheet 45 includes a concave-convex region P where a concave-convex shape is formed respectively. Here, "the concave-convex shape" denotes a concave-convex shape which is formed at a sheet such as nonwoven with concave-convex process such as embossing process. "The concave-convex shape" does not include a fine concave-convex shape (a concave-convex shape in a micro sense) formed at a sheet before performing the concave-convex process. According to absorbent articles respectively with the absorbent members 4C to 4F, it is possible to obtain similar effects to the abovementioned embodiments.

As illustrated in Fig. 5(a), in the absorbent member 4C, the concave-convex region P is formed at the entire area of the upper core wrap sheet 45 and the apparent thickness t1 of the concave-convex region P is larger than the thickness t2 of the lower core wrap sheet 46. That is, the concave-convex region P is the thick region having a thickness (apparent thickness) which is larger than the lower core wrap sheet 46.

As illustrated in Figs. 5(b), 5(c) and 5(d), in the absorbent members 4D, 4E and 4F, a part of the upper core wrap sheet 45, specifically a center part of the upper core wrap sheet 45 in the lateral direction Y, is formed as the concave-convex region P (the abovementioned thick region) over the entire length of the longitudinal direction X respectively. Other regions of the upper core wrap sheet 45 (both side portions in the lateral direction Y) in the absorbent members 4D, 4E and 4F are formed as flat regions Q where a concave-convex shape is not formed. Except for that the upper core wrap sheet 45 has the concave-convex region P, the absorbent member 4D has the same basic structure as the abovementioned absorbent member 4 (see Figs. 2 and 3) and the absorbent member 4E has the same basic structure as the abovementioned absorbent member 4B (see Fig. 4(b)).

As illustrated in Fig. 5(d), in the absorbent member 4F, the lower core wrap sheet 46 is integrated with the upper core wrap sheet 45 as being continuously arranged to form a single core wrap sheet 47 with both the sheets 45, 46 as a whole. In this manner, the upper core wrap sheet and the lower core wrap sheet according to the present invention may be formed by folding a single continuous core wrap sheet along the absorbent core 40. The single core wrap sheet 47 covers the entire area of the skin-facing face of the absorbent core 40 and is extended outward in the lateral direction Y from both the side edge portions of the absorbent core 40 along the longitudinal direction X. The extended portions are rolled down to the non-skin-facing face of the absorbent core 40 and cover the entire area of the non-skin-facing face. The portion which covers the skin-facing face of the absorbent core 40 (the center portion in the lateral direction Y) is the upper core wrap sheet 45 and the portion which covers the non-skin-facing face of the absorbent core 40 (both bilateral side portions in the lateral direction Y) is the lower core wrap sheet 46.

In the upper core wrap sheet 45 which has the concave-convex region P and the flat region Q, the thickness (apparent thickness) of the concave-convex region P is only required to be larger than the thickness of the lower core wrap sheet 46 at least. That is, it is only required that the concave-convex region P is the thick region. Here, it does not matter whether the thickness of the flat region Q is larger or smaller than the thickness of the lower core wrap sheet 46. Further, the concave-convex region P exists over the absorbent core 40 in a range of 30% to 100% of the entire width (entire length in the lateral direction Y) preferably and in a range of 50% to 100% more preferably.

A known solid processing method used for providing a concave-convex shape to a sheet in the art can be used as a method to form the concave-convex region P (concave-convex shape) at the upper core wrap sheet 45. Examples thereof include embossing process such as embossing process with heat and/or pressure and ultrasonic embossing process, tooth groove process for a sheet using tooth groove rollers (a pair of gear rollers with tooth grooves engaged like gears) (e.g., see JP 2009-201964A, JP 2009-50538A, JP 2009-160032A and the like), creping process (e.g., see JP 2004-305771A, JP 8-126663 A, JP 8-260328A and the like), and strain-free process (e.g., see JP 8-502181A, JP 10-502423A, JP 2002-320640A, JP 2006-320730A and the like).

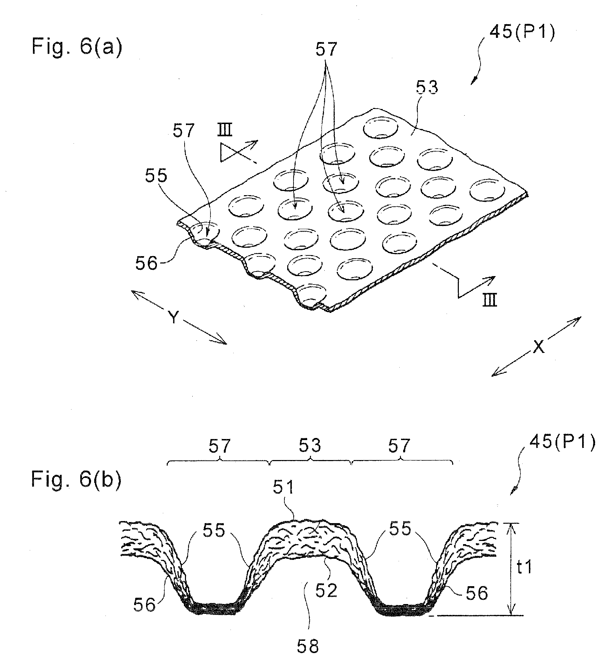

Fig. 6 illustrates an example of the concave-convex region P of the upper core wrap sheet 45. A concave-convex region P1 illustrated in Fig. 6 includes top face portions (convex portions) 53 which are contacted to a skin at a front face 51 side and concave portions 57 respectively with a front face 55 side concaved and a back face 56 side protruded toward the absorbent core 40 (not illustrated in Fig. 6) side. The concave portions 57 are formed by embossing process and are arranged in a staggered pattern at the concave-convex region P1 as illustrated in Fig. 6(a). The front faces 51 of the top face portions 53 and the front faces 55 of the concave portions 57 form one face of the upper core wrap sheet 45 which forms the skin-facing face. Back faces 52 of the top face portions 53 and the back faces 56 of the concave portions 57 form the other face of the upper core wrap sheet 45 which forms the non-skin-facing face. As illustrated in Fig. 6(b), space portions 58 in which structural fibers of the sheet 45 do not exist are formed below the top face portions 53 (between the top face portions 53 and the absorbent core 40 (not illustrated in Fig. 6)). When the space portions 58 are formed between the absorbent core and the convex portions (top face portions 53) which structure the concave-convex region P1 of the upper core wrap sheet 45 as described above, liquid absorption of absorbent polymer existing at positions of the absorbent core below the space portions 58 and expansion of the absorbent polymer thereby are facilitated and leakage prevention is further improved.

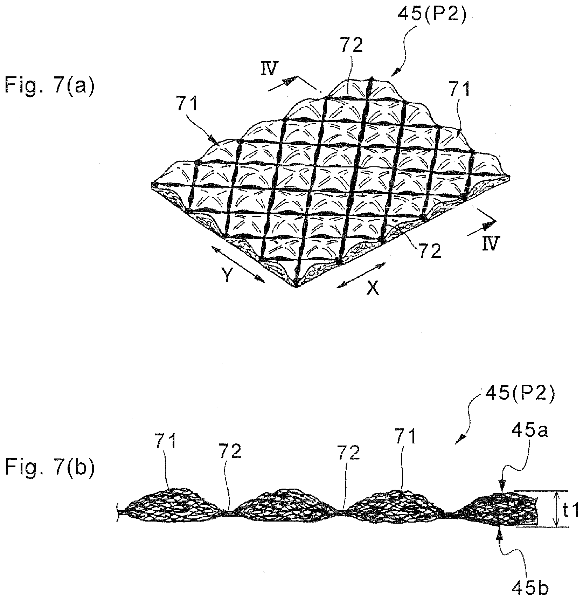

Fig. 7 illustrates another example of the concave-convex region P of the upper core wrap sheet 45. In a concave-convex region P2 illustrated in Fig. 7, a non-skin-facing face 45b thereof is approximately flat and a skin-facing face 45a is formed as a concave-convex shape having a number of convex portions 71 and concave portions 72. The concave portions 72 are structured with embossed portions formed by embossing process. The embossed portion is a portion where constituent fibers of the sheet 45 are joined by heat and/or pressure. The convex portion 71 is located among the concave portions 72. The inside of the convex portions 71 is filled with constituent fibers of the sheet 45. The concave portions 72 are structured with substantially continuous straight lines and are formed so that a plurality of the lines forms a rhombic lattice.

Not being limited to the abovementioned embodiments, the present invention may be appropriately modified without departing from the scope of the present invention. For example, although the sublayer sheet 5 is arranged from the front section B to the crotch section C in the abovementioned embodiment, the arrangement position of the sublayer sheet 5 is not specifically limited. For example, it is possible to be arranged from the rear section A to the crotch section C or to be arranged only at the crotch section C. Alternatively, the sublayer sheet 5 may not be arranged.

Further, other than an open-style type disposable diaper with a fastening tape, the absorbent article of the present invention may be an underpants type disposable diaper which is previously formed in an underpants shape, an absorbent pad, a sanitary napkin, or the like. In relation to the abovementioned embodiments, additional subjects (absorbent articles) are further disclosed.

### Examples

In the following, the present invention will be described more specifically with examples. Here, the present invention is not limited to such examples.

### [Example 1]

A sample for example 1 was prepared as an open-style type disposable diaper having a basic structure being similar to the diaper 1 illustrated in Figs. 1 and 2 except for that the sublayer sheet was not included. The topsheet adopted air-through nonwoven having a basis weight of 25 g/m² with constituent fibers being PET, PP and PE. The backsheet adopted a PE-made resin film having a basis weight of 20 g/m². The absorbent core was structured using absorbent polymer (SAP, IM930 manufactured by San-dia Polymer Corporation) without using fibers and a basis weight of the absorbent core (a dispersal amount of the absorbent polymer) was set to 75 g/m². The upper core wrap sheet adopted hydrophilic SMMS nonwoven having a thickness of 0.2 mm (a basis weight of 15 g/m²), water passing time of 1 second with a basis weight ratio of the respective layers (S : M : M : S) being "6.5 : 1 : 1 : 6.5". The lower core wrap sheet adopted hydrophilic SMMS nonwoven having a thickness of 0.1 mm (a basis weight of 10 g/m²) and water passing time of 1 second with a basis weight ratio of the respective layers (S : M : M : S) being "4 : 1 : 1 : 4". For preparing the absorbent member, mixture of absorbent polymer and adhesive was dispersed onto one face of the lower core wrap sheet and the upper core wrap sheet with adhesive previously applied to one face thereof was overlapped to the dispersed face. The entire length of the absorbent member in the longitudinal direction was 380 mm, the entire length (maximum length) thereof in the lateral direction was 120 mm, and the thickness thereof with no load was 1.3 mm.

### [Example 2]

A sample for example 2 was prepared as an open-style type disposable diaper similarly to example 1 except for that the lower core wrap sheet adopted hydrophobic SMMS nonwoven having a thickness of 0.1 mm (a basis weight of 10 g/m²) and water passing time of 63 seconds with a basis weight ratio of the respective layers (S : M : M : S) being "4 : 1 : 1 : 4".

### [Comparison examples 1 and 2]

Samples for comparison examples 1 and 2 were prepared as an open-style type disposable diaper similarly to example 1 except for that thicknesses (basis weights) of the upper core wrap sheet and the lower core wrap sheet were equaled.

### [Comparison example 3]

A sample for comparison example 3 was prepared as an open-style type disposable diaper similarly to comparison example 1 except for that the sublayer sheet was arranged between the topsheet and the absorbent member as being hydrophilic SMMS nonwoven having a thickness of 0.1 mm (a basis weight of 10 g/m²) with a basis weight ratio of the respective layers (S : M : M : S) being "4 : 1 : 1 : 4". The sublayer sheet is arranged to the front side of the absorbent member.

### [Comparison example 4]

A sample for comparison example 4 was prepared as an open-style type disposable diaper similarly to example 1 except for interchanging thicknesses (basis weights) of the upper core wrap sheet and the lower core wrap sheet.

### [Comparison example 5]

A sample for comparison example 5 was prepared as an open-style type disposable diaper similarly to example 1 except for that the absorbent core adopted an absorbent core substantially containing hydrophilic fibers (cellulose fibers) as well as absorbent polymer (SAP) (flap pulp (NBKP) web with 50 g/m² was overlapped on SAP with 75 g/m²).

### [Evaluation]

Liquid return amounts were measured with a method described below using samples (disposable diapers) of examples and comparison examples. The result thereof is indicated below in table 1. The extent of the liquid return amount is closely related to absorption performance of a disposable diaper, especially, to a dry feeling at the skin-facing face (topsheet). The less liquid return amount provides higher rating with higher absorption performance of a disposable diaper as being superior in dry feeling.

### <Liquid return amount>

An acrylic plate with a cylindrical inlet portion is placed on a topsheet at the center part of an absorbent member in a state that a disposable diaper being a measurement target is planarly expanded and is fixed on a horizontal surface with the skin-facing face (topsheet) faced upward. The inlet portion arranged at the acrylic plate is cylindrical having an inner diameter of 20 mm. A penetration hole having an inner diameter of 10 mm which provides communication between the inside of the cylindrical inlet portion and a face of the acrylic plate faced to the topsheet is formed at the acrylic plate while the center axis line of the cylindrical inlet portion is matched with the center thereof in the longitudinal direction and the lateral direction. Subsequently, the acrylic plate is arranged so that the center axis of the cylindrical inlet portion is matched with the center part of the absorbent member in plane view. Then, artificial urine of 30 g is poured from the cylindrical inlet portion and is to be absorbed by the disposable diaper. After the artificial urine is powered, the acrylic plate is removed. After one minute of pouring, twenty sheets of filter paper (hard filter paper 4A manufactured by ADVANTEC CO., LTD) of 10 centimeters square are overlapped and placed on the poured position on the skin-facing face (topsheet) of the disposable diaper. Then, pressurization is performed for thirty seconds with a load of 0.7 kPa or below 0.1 kPa. Thus, artificial urine liquid-returned with pressurization is absorbed by the filter paper. A weight of the filter paper increased with the absorption is measured and the difference against a weight of the filter paper before the absorption is taken as the liquid return amount.

**[Table 1]**

| | | Examples | | Comparison examples | | | | |
|---|---|---|---|---|---|---|---|---|
| | | 1 | 2 | 1 | 2 | 3 | 4 | 5 |
| Upper core wrap sheet | Type | SMMS nonwoven | SMMS nonwoven | SMMS nonwoven | SMMS nonwoven | SMMS nonwoven | SMMS nonwoven | SMMS nonwoven |
| | Water passing time (second) | 1 | 1 | 1 | 1 | 1 | 1 | 1 |
| | Thickness (mm) | 0.2 | 0.2 | 0.1 | 0.2 | 0.1 | 0.1 | 0.2 |
| | Basis weight (g/m²) | 15 | 15 | 10 | 15 | 10 | 10 | 15 |
| Lower core wrap sheet | Type | SMMS nonwoven | SMMS nonwoven | SMMS nonwoven | SMMS nonwoven | SMMS nonwoven | SMMS nonwoven | SMMS nonwoven |
| | Water passing time (second) | 1 | 63 | 1 | 1 | 1 | 1 | 1 |
| | Thickness (mm) | 0.1 | 0.1 | 0.1 | 0.2 | 0.1 | 0.2 | 0.1 |
| | Basis weight (g/m²) | 10 | 10 | 10 | 15 | 10 | 15 | 10 |
| Absorbent core | Constituent | SAP only | SAP only | SAP only | SAP only | SAP only | SAP only | SAP and pulp |
| | Basis weight (g/m²) | 75 | 75 | 75 | 75 | 75 | 75 | 75+50 |
| Basis weight of absorbent member (g/m²) | | 100 | 100 | 95 | 105 | 95 | 100 | 150 |
| Sublayer sheet | | Absent | Absent | Absent | Absent | Present | Absent | Absent |
| Liquid return amount (g) | Load of 0.7 kPa. | 1.6 | 1.9 | 2.0 | 2.5 | 2.0 | 3.1 | 3.5 |
| | Load below 0.1 kPa | 0.1 | 0.1 | 0.8 | 0.2 | 0.5 | 0.8 | 0.5 |

As clearly seen from the result indicated in table 1, it is revealed that the disposable diapers of the examples (the present invention) have less liquid return amounts in both cases of the load at measuring the liquid return amount being 0.7 kPa and being below 0.1 kPa. The load of 0.7 kPa corresponds to a load applied to a diaper in a state that a young month age infant wearing the diaper lies on his/her back. When the liquid return amount is below 2 g at the load of 0.7 kPa, liquid is not moved along a skin in a case that liquid exists between a skin of a wearer and the topsheet. Further, the load below 0.1 kPa reflects a feeling of touching a surface (skin-facing face) of a diaper without substantially little pressurization to the diaper. When the liquid return amount is below 0.2 g at the load below 0.1 kPa, a wearer hardly feels wet. In contrast, any of the respective comparison examples does not satisfy both conditions that liquid is not moved along a skin and that a wet feeling does not exist at a surface of a diaper. In comparison examples 1 to 4, the upper core wrap sheet does not include the thick region having a larger thickness than the lower core wrap sheet. Further, in comparison example 5, the absorbent core substantially contains hydrophilic fibers. The comparison examples are different from the examples in the abovementioned points. The above reveals that the following is important for improving a dry feeling at the skin-facing face of an absorbent article, that is, 1) the upper core wrap sheet includes the thick region having a larger thickness than the lower core wrap sheet and 2) the absorbent core does not substantially contain hydrophilic fibers.

## Claims

1. An absorbent article (1), comprising:
a topsheet (2) which forms a skin-facing face;
a backsheet (3) which forms a non-skin-facing face; and
an absorbent member (4) which is arranged between both the sheets (2, 3),
**characterized in that** the absorbent member (4) is structured to include an absorbent core (40) which contains absorbent polymer without substantially containing hydrophilic fibers, an upper core wrap sheet (45) which covers a skin-facing face of the absorbent core (40), and a lower core wrap sheet (46) which covers a non-skin-facing face of the absorbent core (40), and
the upper core wrap sheet (45) includes a thick region having a larger thickness than the lower core wrap sheet (46).

2. The absorbent article (1) according to claim 1, wherein content of the hydrophilic fibers of the absorbent core (40) is 5% or less by mass.

3. The absorbent article (1) according to claim 1, wherein the absorbent core (40) does not contain the hydrophilic fibers at all.

4. The absorbent article (1) according to any one of claims 1 to 3, wherein the upper core wrap sheet (45) includes a concave-convex region (P) where a concave-convex shape is formed.

5. The absorbent article (1) according to any one of claims 1 to 4,
wherein each of the upper core wrap sheet (45) and the lower core wrap sheet (46) is formed of a laminated body with one or more of spunbond nonwoven layers and one or more of meltblown nonwoven layers,
a basis weight of the spunbond nonwoven layer is larger than that of the meltblown nonwoven layer in the upper core wrap sheet (45), and
a basis weight of the meltblown nonwoven layer is larger than that of the spunbond nonwoven layer in the lower core wrap sheet (46).

6. The absorbent article (1) according to claim 5, wherein the laminated body is nonwoven integrated in a state that two layers of the meltblown nonwoven layers are placed between two layers of the spunbond nonwoven layers.

7. The absorbent article (1) according to any one of claims 1 to 6, wherein a hydrophilic degree of the upper core wrap sheet (45) is higher than that of the lower core wrap sheet (46).

8. The absorbent article (1) according to any one of claims 1 to 7, wherein the lower core wrap sheet (46) has hydrophobicity.

9. The absorbent article (1) according to any one of claims 1 to 8, wherein a thickness of the thick region of the upper core wrap sheet (45) is twice or more of a thickness of the lower core wrap sheet (46).

10. The absorbent article (1) according to any one of claims 1 to 9 used for a disposable diaper.

11. The absorbent article (1) according to any one of claims 1 to 9 used for an open-style type disposable diaper with a fastening tape (8).

## Patentansprüche

1. Absorptionsartikel (1), der aufweist:
ein Topsheet (2), das eine zur Haut weisende Fläche bildet;
ein Backsheet (3), das eine nicht zur Haut weisende Fläche bildet; und
ein Absorptionsteil (4), das zwischen beiden der Bahnen (2, 3) angeordnet ist,
**dadurch gekennzeichnet, dass** das Absorptionsteil (4) so aufgebaut ist, dass es aufweist: einen Absorptionskern (40), der ein absorptionsfähiges Polymer enthält, ohne im Wesentlichen hydrophile Fasern zu enthalten, eine obere Kemhüllenbahn (45), die eine zur Haut weisende Fläche des Absorptionskerns (40) abdeckt, und eine untere Kernhüllenbahn (46), die eine nicht zur Haut weisende Fläche des Absorptionskerns (40) abdeckt, und
die obere Kernhüllenbahn (45) einen dicken Bereich mit einer größeren Dicke als die untere Kernhüllenbahn (46) aufweist.

2. Absorptionsartikel (1) nach Anspruch 1, wobei der Gehalt der hydrophilen Fasern des Absorptionskerns (40) 5 Masse-% oder weniger beträgt.

3. Absorptionsartikel (1) nach Anspruch 1, wobei der Absorptionskern (40) überhaupt keine hydrophilen Fasern enthält.

4. Absorptionsartikel (1) nach einem der Ansprüche 1 bis 3, wobei die obere Kernhüllenbahn (45) einen konkavkonvexen Bereich (P) aufweist, in dem eine konkavkonvexe Form gebildet ist.

5. Absorptionsartikel (1) nach einem der Ansprüche 1 bis 4, wobei die obere Kernhüllenbahn (45) und die untere Kernhüllenbahn (46) jeweils aus einem laminierten Körper mit einer oder mehreren Spundbond-Vliesschichten und einer oder mehreren Meltblown-Vliesschichten gebildet sind,
ein Flächengewicht der Spundbond-Vliesschicht größer als das der Meltblown-Vliesschicht in der oberen Kernhüllenbahn (45) ist und
ein Flächengewicht der Meltblown-Vliesschicht größer als das der Spundbond-Vliesschicht in der unteren Kernhüllenbahn (46) ist.

6. Absorptionsartikel (1) nach Anspruch 5, wobei der laminierte Körper Vlies ist, das in einem Zustand integriert ist, in dem zwei Schichten der Meltblown-Vliesschichten zwischen zwei Schichten der Spundbond-Vliesschichten platziert sind.

7. Absorptionsartikel (1) nach einem der Ansprüche 1 bis 6, wobei ein hydrophiler Grad der oberen Kernhüllenbahn (45) höher als der der unteren Kernhüllenbahn (46) ist.

8. Absorptionsartikel (1) nach einem der Ansprüche 1 bis 7, wobei die untere Kernhüllenbahn (46) Hydrophobie hat.

9. Absorptionsartikel (1) nach einem der Ansprüche 1 bis 8, wobei eine Dicke des dicken Bereichs der oberen Kernhüllenbahn (45) mindestens doppelt so groß wie eine Dicke der unteren Kernhüllenbahn (46) ist.

10. Absorptionsartikel (1) nach einem der Ansprüche 1 bis 9, der für eine Wegwerfwindel verwendet wird.

11. Absorptionsartikel (1) nach einem der Ansprüche 1 bis 9, der für eine Open-Style-Wegwerfwindel mit einem Befestigungsband (8) verwendet wird.

## Revendications

1. Article absorbant (1), comprenant :
une feuille de dessus (2) qui forme une face de contact avec la peau ;
une feuille de support (3) qui forme une face de non contact avec la peau ; et
un élément absorbant (4) qui est agencé entre les deux feuilles (2, 3),
**caractérisé en ce que** l'élément absorbant (4) est structuré pour comporter un cœur absorbant (40) qui contient un polymère absorbant sans sensiblement contenir de fibres hydrophiles, une feuille d'enveloppage de cœur supérieure (45) qui couvre une face de contact avec la peau du cœur absorbant (40), et une feuille d'enveloppage de cœur inférieure (46) qui couvre une face de non contact avec la peau du cœur absorbant (40), et
la feuille d'enveloppage de cœur supérieure (45) comporte une région épaisse ayant une épaisseur plus importante que la feuille d'enveloppage de cœur inférieure (46).

2. Article absorbant (1) selon la revendication 1, dans lequel la teneur en fibres hydrophiles du cœur absorbant (40) est de 5 % ou moins en masse.

3. Article absorbant (1) selon la revendication 1, dans lequel le cœur absorbant (40) ne contient pas de fibres hydrophiles du tout.

4. Article absorbant (1) selon l'une quelconque des revendications 1 à 3, dans lequel la feuille d'enveloppage de cœur supérieure (45) comprend une région concavo-convexe (P) où une forme concavo-convexe est formée.

5. Article absorbant (1) selon l'une quelconque des revendications 1 à 4,
dans lequel chacune de la feuille d'enveloppage de cœur supérieure (45) et de la feuille d'enveloppage de cœur inférieure (46) est formée d'un corps stratifié avec une ou plusieurs couches non tissées filées liées et une ou plusieurs couches non tissées soufflées à l'état fondu,
un grammage de la couche non tissée filée liée est supérieur à celui de la couche non tissée soufflée à l'état fondu dans la feuille d'enveloppage de cœur supérieure (45), et
un grammage de la couche non tissée soufflée à l'état fondu est supérieur à celui de la couche non tissée filée liée dans la feuille d'enveloppage de cœur inférieure (46).

6. Article absorbant (1) selon la revendication 5, dans lequel le corps stratifié est non tissé intégré dans un état dans lequel deux couches des couches non tissées soufflées à l'état fondu sont placées entre deux couches des couches non tissées filées liées.

7. Article absorbant (1) selon l'une quelconque des revendications 1 à 6, dans lequel un degré d'hydrophilie de la feuille d'enveloppage de cœur supérieure (45) est supérieur à celui de la feuille d'enveloppage de cœur inférieure (46).

8. Article absorbant (1) selon l'une quelconque des revendications 1 à 7, dans lequel la feuille d'enveloppage de cœur inférieure (46) présente une hydrophobicité.

9. Article absorbant (1) selon l'une quelconque des revendications 1 à 8, dans lequel une épaisseur de la région épaisse de la feuille d'enveloppage de cœur supérieure (45) est deux fois ou plus une épaisseur de la feuille d'enveloppage de cœur inférieure (46).

10. Article absorbant (1) selon l'une quelconque des revendications 1 à 9 utilisé pour une couche jetable.

11. Article absorbant (1) selon l'une quelconque des revendications 1 à 9 utilisé pour une couche jetable de type style ouvert avec une bande de fixation (8) .
